# EUROPEAN PATENT SPECIFICATION

(11) **EP 4 263 485 B1**
(45) Date of publication and mention of the grant of the patent: **18.09.2024**
(21) Application number: 21831419.3
(22) Date of filing: 02.12.2021
(51) Int. Cl.: C07C 51/25, C07C 57/045

(54) **LIQUID-PHASE OXIDATION OF ACROLEIN WITH GOLD BASED CATALYSTS**
FLÜSSIGPHASENOXIDATION VON ACROLEIN MIT GOLDBASIERTEN KATALYSATOREN
OXYDATION EN PHASE LIQUIDE DE L'ACROLÉINE AVEC DES CATALYSEURS À BASE D'OR

(30) Priority: 18.12.2020 US 202063127652 P
(43) Date of publication of application: 25.10.2023
(73) Proprietor: Dow Global Technologies LLC, Midland, MI 48674 (US); Rohm and Haas Company, Collegeville, PA 19426 (US)
(72) Inventor: HOUSER, Jing L., Midland, Michigan 48667 (US); KRAPCHETOV, Dmitry A., Collegeville, Pennsylvania 19426 (US)
(74) Representative: Jewell, Catherine Mary
(86) International application number: PCT/US2021/061512
(87) International publication number: WO 2022/132442

(56) References cited:
- JP-A- 2007 301 503
- US-A- 3 736 354
- US-A- 4 107 204
- OHKATSU YASUKAZU ET AL: "The Liquid Phase Oxidation of Acrolein. V. Effects of Water on the Oxidation of Acrolein", BULLETIN OF THE CHEMICAL SOCIETY OF JAPAN, vol. 40, no. 9, 1 September 1967 (1967-09-01), JP, pages 2116 - 2121, XP055900947, ISSN: 0009-2673, Retrieved from the Internet <URL:https://www.journal.csj.jp/doi/pdf/10.1246/bcsj.40.2116> DOI: 10.1246/bcsj.40.2116

## Description

### BACKGROUND OF THE INVENTION

The invention relates to a method for preparing acrylic acid from liquid-phase oxidation of acrolein using a gold based catalyst.

Acrylic acid, methacrylic acid, and their esters are important chemicals with widespread uses for the manufacture of various polymers, coatings, adhesives, elastomers, and other products.

Traditionally, acrylic acid has been prepared by the gas-phase oxidation of propylene, where the propylene may first be oxidized to form acrolein, which is subsequently converted to acrylic acid.

Liquid-phase oxidation is an attractive alternative to gas-phase oxidation because liquid-phase oxidation is expected to require milder conditions and smaller equipment.

Attempts have been made to prepare acrylic acid by liquid-phase oxidation. For example, Yasukazu et al. "The Liquid Phase Oxidation of Acrolein V: Effects of Water on the Oxidation of Acrolein," Bulletin of the Chemical Society of Japan, 1967, 40, 2116-2121, explored the effects of water in the liquid-phase oxidation of acrolein with cobalt-based catalysts.

There is a need for an improved process for the liquid-phase oxidation of acrolein to produce acrylic acid.

### SUMMARY OF THE INVENTION

The present invention is directed to a method for preparing acrylic acid from acrolein. The method comprises contacting a liquid mixture comprising acrolein and water in the presence of oxygen with a heterogeneous catalyst comprising a support and gold, wherein the support comprises an oxide selected from γ-, δ-, or θ-alumina, magnesia, titania, zirconia, hafnia, vanadia, niobium oxide, tantalum oxide, ceria, yttria, lanthanum oxide, zinc oxide or a combination thereof.

### DETAILED DESCRIPTION OF THE INVENTION

The present invention is directed to the liquid-phase oxidation of acrolein to form acrylic acid.

In the method of the present invention, a liquid mixture comprising acrolein and water is contacted with a heterogeneous catalyst in the presence of oxygen.

The heterogeneous catalyst comprises gold on a support. The support comprises a particle of an oxide selected from γ-, δ-, or θ-alumina, magnesia, titania, zirconia, hafnia, vanadia, niobium oxide, tantalum oxide, ceria, yttria, lanthanum oxide, zinc oxide or a combination thereof. Preferably, the support comprises γ-, δ-, or θ-alumina, titania, ceria, zinc oxide or a combination thereof. More preferably, the support comprises titania.

Preferably, the support does not comprise undoped silica or silicon carbide. For example, the support may comprise ceria doped silica, but may not comprise undoped silica.

Preferably, in portions of the catalyst comprising the noble metal, the support has a surface area greater than 10 m²/g, preferably greater than 30 m²/g, preferably greater than 50 m²/g, preferably greater than 100 m²/g, preferably greater than 120 m²/g. In portions of the catalyst which comprise little or no noble metal, the support may have a surface area less than 50 m²/g, preferably less than 20 m²/g.

Preferably, at least 90 wt% of the noble metal(s) is in the outer 50% of catalyst volume (i.e., the volume of an average catalyst particle), preferably the outer 40%, preferably in the outer 35%, preferably in the outer 30%, preferably in the outer 25%. Preferably, the outer volume of any particle shape is calculated for a volume having a constant distance from its inner surface to its outer surface (the surface of the particle), measured along a line perpendicular to the outer surface.

For example, for a spherical particle the outer x% of volume is a spherical shell whose outer surface is the surface of the particle and whose volume is x% of the volume of the entire sphere. Preferably, at least 95 wt% of the noble metal is in the outer volume of the catalyst, preferably at least 97 wt%, preferably at least 99 wt%. Preferably, at least 90 wt% (preferably at least 95 wt%, preferably at least 97 wt%, preferably at least 99 wt%) of the noble metal(s) is within a distance from the surface that is no more than 15% of the catalyst diameter, preferably no more than 10%, preferably no more than 8%, preferably no more than 6%. Distance from the surface is measured along a line which is perpendicular to the surface. The "catalyst center" is the centroid of the catalyst particle, i.e., the mean position of all points in all coordinate directions. A diameter is any linear dimension passing through the catalyst center and the average diameter is the arithmetic mean of all possible diameters.

The aspect ratio is the ratio of the longest to the shortest diameters. Preferably, the aspect ratio of the catalyst particle is no more than 10:1, preferably no more than 5:1, preferably no more than 3:1, preferably no more than 2:1, preferably no more than 1.5:1, preferably no more than 1.1:1. Preferred shapes for the catalyst particle include spheres, cylinders, rectangular solids, rings, multi-lobed shapes (e.g., cloverleaf cross section), shapes having multiple holes and "wagon wheels;" preferably spheres. Irregular shapes may also be used.

Preferably, the average diameter of the catalyst particle is at least 200 microns, preferably at least 400 microns, more preferably at least 600 microns, and even preferably at least 800 microns. The average diameter of the catalyst particles is preferably no more than 30 mm, more preferably no more than 20 mm, and even more preferably no more than 10 mm. The average diameter of the support and the average diameter of the final catalyst particle are not significantly different.

Preferably, the amount of gold as a percentage of the gold and the support is from 0.2 to 5 wt%, preferably at least 0.5 wt%, preferably at least 0.8 wt%, preferably at least 1 wt%, preferably at least 1.2 wt%; preferably no more than 4 wt%, preferably no more than 3 wt%, preferably no more than 2.5 wt%.

Preferably, the catalyst is produced by precipitating the gold from an aqueous solution of a gold salt in the presence of the support. In one particular case of the present disclosure (not forming part of the claimed invention), the catalyst is produced by incipient wetness in which an aqueous solution of a suitable gold precursor salt is added to a porous inorganic oxide such that the pores are filled with the solution and the water is then removed by drying. Preferred salts include tetrachloroauric acid, sodium aurothiosulfate, sodium aurothiomalate, and gold hydroxide. The resulting material is then converted into a finished catalyst by calcination, reduction, or other treatments known to those skilled in the art to decompose the gold salts into metals or metal oxides. Preferably, a C₂-C₁₈ thiol comprising at least one hydroxyl or carboxylic acid substituent is present in the solution. Preferably, the C₂-C₁₈ thiol comprising at least one hydroxyl or carboxylic acid substituent has from 2 to 12 carbon atoms, preferably 2 to 8, preferably 3 to 6. Preferably, the thiol compound comprises no more than 4 total hydroxyl and carboxylic acid groups, preferably no more than 3, preferably no more than 2. Preferably, the thiol compound has no more than 2 thiol groups, preferably no more than one. If the thiol compound comprises carboxylic acid substituents, they may be present in the acid form, conjugate base form or a mixture thereof. The thiol component also may be present either in its thiol (acid) form or its conjugate base (thiolate) form. Especially preferred thiol compounds include thiomalic acid, 3-mercaptopropionic acid, thioglycolic acid, 2-mercaptoethanol and 1-thioglycerol, including their conjugate bases.

In one particular case of the present disclosure (not forming part of the claimed invention), the catalyst is produced by deposition precipitation in which a porous inorganic oxide is immersed in an aqueous solution containing a suitable gold precursor salt and that salt is then made to interact with the surface of the inorganic oxide by adjusting the pH of the solution. The resulting treated solid is then recovered (e.g. by filtration) and then converted into a finished catalyst by calcination, reduction, or other treatments known to those skilled in the art to decompose the gold salts into metals or metal oxides.

The catalyst particles are preferably contained in a catalyst bed and typically are held in place by solid walls and by screens or catalyst support grids. In some configurations, the screens or grids are on opposite ends of the catalyst bed and the solid walls are on the side(s), although in some configurations the catalyst bed may be enclosed entirely by screens. Preferred shapes for the catalyst bed include a cylinder, a rectangular solid and a cylindrical shell; preferably a cylinder.

The liquid mixture comprises acrolein and water. The water may be present in the liquid mixture in an amount ranging from 1 to 95 wt% relative to the total weight of the liquid mixture. Preferably, the water is present in an amount of at least 5 wt%, and more preferably at least 10 wt%, and still more preferably at least 20 wt% relative to the total weight of the liquid mixture. Preferably, the water is present in an amount less than 80 wt% relative to the total weight of the liquid mixture.

The acrolein may be present in the liquid mixture in an amount ranging from 2 to 70 wt% relative to the total weight of the liquid mixture. Preferably, the acrolein is present in an amount of at least 3 wt%, more preferably at least 5 wt%, and even more preferably at least 10 wt% relative to the total weight of the liquid mixture. Preferably, the acrolein is present in an amount less than or equal to 50 wt% relative to the total weight of the liquid mixture, such as, for example, less than or equal to 40 wt% or less than or equal to 30 wt% relative to the total weight of the liquid mixture.

The liquid mixture may further comprise an organic solvent. Preferably, the organic solvent has an oxygen solubility greater than that of water (8.2 ppm at 25 °C and 1 atm (101 kPa)). For example, the organic solvent may have an oxygen solubility greater than 100 ppm at 25 °C and 1 atm (101 kPa). More preferably, the organic solvent has an oxygen solubility greater than 150 ppm at 25 °C and 1 atm (101 kPa), and even more preferably greater than 300 ppm at 25 °C and 1 atm (101 kPa). Without wishing to be bound by theory, it is believed that the addition of an organic solvent with an oxygen solubility higher than that of water may help increase the capability of the liquid mixture to supply oxygen for the reaction.

Examples of the organic solvents that may be present in the liquid mixture include, but are not limited to acetonitrile, toluene, o-xylene, hexane, octane, N,N-dimethyl formamide, Fluorinert^{™} FC-770, dichloroethane, acetone, and diglyme (bis(2-methoxyethyl) ether). Preferably, the organic solvent comprises acetone, N,N-dimethyl formamide, or diglyme. Even more preferably, the organic solvent comprises diglyme.

The organic solvent may be miscible with water. As defined herein, an organic solvent is miscible with water if a mixture forms a homogeneous solution.

In the method of the present invention, the liquid mixture is contacted with the heterogeneous catalyst in the presence of oxygen. For example, the oxygen may be supplied to the reaction using air or oxygen, with or without an inert gas.

The reaction may take place, for example, at a temperature ranging from 40 to 120 °C. Preferably, the temperature is at least 50 °C, and more preferably at least 60 °C. The temperature is preferably less than 110 °C, and more preferably less than 100 °C.

The reaction may take place at a pressure ranging from 0.5 to 100 bar; preferably no more than 80 bar, and more preferably no more than 20 bar.

### EXAMPLES

All examples were run with a 300 ml Parr reactor. All the reactions were run at 80 °C and 100 psig (790 kPa). The gas feed consisted of 8% of O₂ and 92% of N₂. The limited O₂ concentration in the gas feed was to ensure the avoidance of the reactants' and the solvents' flammable zones. 200-500 ppm of hydroquinone (HQ) was used in each run as polymerization inhibitor. 3 g of gold based catalysts with different supports were used for catalyzing the oxidative reaction. The reaction time was 1 hr and 20 min for most of the runs, except that two runs lasted for 2 hr. The final reaction mixtures were analyzed with gas chromatography (GC).

The oxidation of acrolein (ACRL) to produce acrylic acid (AA) was run with diglyme as the solvent (Table 1). The highest AA selectivity achieved was 58.9%. Examining the chromatograms, a major side-product was identified as the acrylic acid dimer (AAD). The results suggest that increasing the concentration of water in the reactant mixture promoted the production rate of AAD more than AA, thus decreasing AA selectivity. Increasing the concentration of ACRL in the reactant mixture had a similar impact. The experiments also suggested that some catalyst supports suppressed the production of AAD and enhanced AA selectivity. Overall, the space time yields of both AA and AAD dropped with increasing AA selectivity.

**Table 1**

| Expt. # | Catalyst | wt% water | wt% diglyme | wt% ACRL | AA Space time yield (mol/kg-cat/hr) | AAD space time yield (mol/kg-cat/hr) | AA Selectivity |
|---|---|---|---|---|---|---|---|
| Ex. 1 | Au/TiO2 | 10.0% | 82.2% | 7.8% | 2.14 | 2.82 | 46.1% |
| Ex. 2 | Au/TiO2 | 16.7% | 75.6% | 7.8% | 2.78 | 3.55 | 41.8% |
| Ex. 3 | Au/TiO2 | 22.2% | 70.0% | 7.8% | 2.89 | 4.11 | 40.7% |
| Ex. 4 | Au/CeO2 doped SiO2 | 22.2% | 70.0% | 7.8% | 2.33 | 1.27 | 58.9% |
| Ex. 5 | Pilot-scale Au/Ti-Q10 | 22.2% | 70.0% | 7.8% | 2.04 | 1.66 | 57.3% |
| Ex. 6 | Au/TiO2 | 22.2% | 57.8% | 20.0% | 4.03 | 6.42 | 36.7% |

Unless otherwise indicated by the context of the specification, all amounts, ratios and percentages are by weight, and all test methods are current as of the filing date of this disclosure. The articles "a", "an" and "the" each refer to one or more.

Further, any ranges and subranges relied upon in describing various embodiments of the present invention independently and collectively fall within the scope of the appended claims, and are understood to describe and contemplate all ranges including whole and/or fractional values therein, even if such values are not expressly written herein. One of skill in the art readily recognizes that the enumerated ranges and subranges sufficiently describe and enable various embodiments of the present invention, and such ranges and subranges may be further delineated into relevant halves, thirds, quarters, fifths, and so on. As just one example, a range "of from 0.1 to 0.9" may be further delineated into a lower third, i.e., from 0.1 to 0.3, a middle third, i.e., from 0.4 to 0.6, and an upper third, i.e., from 0.7 to 0.9, which individually and collectively are within the scope of the appended claims, and may be relied upon individually and/or collectively and provide adequate support for specific embodiments within the scope of the appended claims. In addition, with respect to the language which defines or modifies a range, such as "at least," "greater than," "less than," "no more than," and the like, it is to be understood that such language includes subranges and/or an upper or lower limit. As another example, a range of "at least 10" inherently includes a subrange of from at least 10 to 35, a subrange of from at least 10 to 25, a subrange of from 25 to 35, and so on, and each subrange may be relied upon individually and/or collectively and provides adequate support for specific embodiments within the scope of the appended claims. Finally, an individual number within a disclosed range may be relied upon and provides adequate support for specific embodiments within the scope of the appended claims. For example, a range "of from 1 to 9" includes various individual integers, such as 3, as well as individual numbers including a decimal point (or fraction), such as 4.1, which may be relied upon and provide adequate support for specific embodiments within the scope of the appended claims.

The term "composition," as used herein, includes material(s) which comprise the composition, as well as reaction products and decomposition products formed from the materials of the composition.

The term "comprising," and derivatives thereof, is not intended to exclude the presence of any additional component, step or procedure, whether or not the same is disclosed herein. In order to avoid any doubt, all compositions claimed herein through use of the term "comprising" may include any additional additive, adjuvant, or compound, whether polymeric or otherwise, unless stated to the contrary. In contrast, the term, "consisting essentially of" excludes from the scope of any succeeding recitation any other component, step or procedure, excepting those that are not essential to operability. The term "consisting of" excludes any component, step or procedure not specifically delineated or listed.

## Claims

1. A method for preparing acrylic acid from acrolein comprising:
contacting a liquid mixture comprising acrolein and water in the presence of oxygen with a heterogeneous catalyst comprising a support and gold; wherein the support comprises an oxide selected from γ-, δ-, or θ-alumina, magnesia, titania, zirconia, hafnia, vanadia, niobium oxide, tantalum oxide, ceria, yttria, lanthanum oxide, zinc oxide or a combination thereof.

2. The method of claim 1, wherein the water is present in the liquid mixture in an amount ranging from 1 to 95 wt% relative to the total weight of the liquid mixture.

3. The method of claim 2, wherein the water is present in the liquid mixture in an amount ranging from 20 to 80 wt% relative to the total weight of the liquid mixture.

4. The method of any one of the preceding claims, wherein the liquid mixture further comprises an organic solvent.

5. The method of claim 4, wherein the organic solvent has an oxygen solubility greater than 8.2 ppm at 25 °C and 1 atm (101 kPa).

6. The method of claim 4 or 5, wherein the organic solvent is selected from the group consisting of N,N-dimethylformamide, acetone, and diglyme.

7. The method of claim 6, wherein the organic solvent comprises diglyme.

8. The method of claim 4 or 5, wherein the organic solvent is miscible in water.

9. The method of any one of the preceding claims, wherein the catalyst has an average diameter from 200 microns to 10 mm.

10. The method of any one of the preceding claims, wherein at least 90 wt% of the noble metal is in the outer 50% of catalyst volume.

11. The method of claim 10, wherein at least 95 wt% of the noble metal is in the outer 30% of catalyst volume.

12. The method of any one of the preceding claims, wherein the support is selected from the group consisting of γ-, δ-, or θ-alumina, titania, ceria, and zinc oxide.

13. The method of any one of the preceding claims, wherein the support comprises titania.

14. The method of any one of the preceding claims, wherein the support does not comprise undoped silica or silicon carbide.

## Patentansprüche

1. Verfahren zum Herstellen von Acrylsäure aus Acrolein, umfassend:
Inkontaktbringen einer flüssigen Mischung, die Acrolein und Wasser umfasst, mit einem heterogenen Katalysator, der einen Träger und Gold umfasst, in Gegenwart von Sauerstoff; wobei der Träger ein Oxid umfasst, das aus γ-, δ- oder θ-Aluminiumoxid, Magnesiumoxid, Titandioxid, Zirkoniumdioxid, Hafniumdioxid, Divanadiumpentoxid, Nioboxid, Tantaloxid, Cerdioxid, Yttriumoxid, Lanthanoxid, Zinkoxid oder einer Kombination davon ausgewählt ist.

2. Verfahren nach Anspruch 1, wobei das Wasser in der flüssigen Mischung in einer Menge vorhanden ist, die bezogen auf das Gesamtgewicht der flüssigen Mischung von 1 bis 95 Gew.-% reicht.

3. Verfahren nach Anspruch 2, wobei das Wasser in der flüssigen Mischung in einer Menge vorhanden ist, die bezogen auf das Gesamtgewicht der flüssigen Mischung von 20 bis 80 Gew.-% reicht.

4. Verfahren nach einem der vorstehenden Ansprüche, wobei die flüssige Mischung ferner ein organisches Lösungsmittel umfasst.

5. Verfahren nach Anspruch 4, wobei das organische Lösungsmittel eine Sauerstofflöslichkeit von mehr als 8,2 ppm bei 25 °C und 1 atm (101 kPa) aufweist.

6. Verfahren nach Anspruch 4 oder 5, wobei das organische Lösungsmittel ausgewählt ist aus der Gruppe bestehend aus N,N-Dimethylformamid, Aceton und Diethylenglycoldimethylether.

7. Verfahren nach Anspruch 6, wobei das organische Lösungsmittel Diethylenglycoldimethylether umfasst.

8. Verfahren nach Anspruch 4 oder 5, wobei das organische Lösungsmittel in Wasser mischbar ist.

9. Verfahren nach einem der vorstehenden Ansprüche, wobei der Katalysator einen durchschnittlichen Durchmesser von 200 Mikrometer bis 10 mm aufweist.

10. Verfahren nach einem der vorstehenden Ansprüche, wobei mindestens 90 Gew.-% des Edelmetalls in den äußeren 50 % des Katalysatorvolumens vorliegt.

11. Verfahren nach Anspruch 10, wobei mindestens 95 Gew.-% des Edelmetalls in den äußeren 30 % des Katalysatorvolumens vorliegt.

12. Verfahren nach einem der vorstehenden Ansprüche, wobei der Träger ausgewählt ist aus der Gruppe bestehend aus γ-, δ- oder θ-Aluminiumoxid, Titandioxid, Cerdioxid und Zinkoxid.

13. Verfahren nach einem der vorstehenden Ansprüche, wobei der Träger Titandioxid umfasst.

14. Verfahren nach einem der vorstehenden Ansprüche, wobei der Träger kein undotiertes Siliciumdioxid oder Siliciumcarbid umfasst.

## Revendications

1. Procédé de préparation d'acide acrylique à partir d'acroléine comprenant :
la mise en contact d'un mélange liquide comprenant de l'acroléine et de l'eau en présence d'oxygène avec un catalyseur hétérogène comprenant un support et de l'or ; dans lequel le support comprend un oxyde choisi parmi alumine γ, δ ou θ, magnésie, oxyde de titane, zircone, oxyde d'hafnium, oxyde de vanadium, oxyde de niobium, oxyde de tantale, oxyde de cérium, oxyde d'yttrium, oxyde de lanthane, oxyde de zinc ou une combinaison de ceux-ci.

2. Procédé selon la revendication 1, dans lequel l'eau est présente dans le mélange liquide en une quantité allant de 1 à 95 % en poids par rapport au poids total du mélange liquide.

3. Procédé selon la revendication 2, dans lequel l'eau est présente dans le mélange liquide en une quantité allant de 20 à 80 % en poids par rapport au poids total du mélange liquide.

4. Procédé selon l'une quelconque des revendications précédentes, dans lequel le mélange liquide comprend en outre un solvant organique.

5. Procédé selon la revendication 4, dans lequel le solvant organique a une solubilité dans l'oxygène supérieure à 8,2 ppm à 25 °C et 1 atm (101 kPa).

6. Procédé selon la revendication 4 ou 5, dans lequel le solvant organique est choisi dans le groupe constitué de N,N-diméthylformamide, acétone et diglyme.

7. Procédé selon la revendication 6, dans lequel le solvant organique comprend du diglyme.

8. Procédé selon la revendication 4 ou 5, dans lequel le solvant organique est miscible dans l'eau.

9. Procédé selon l'une quelconque des revendications précédentes, dans lequel le catalyseur a un diamètre moyen allant de 200 micromètres à 10 mm.

10. Procédé selon l'une quelconque des revendications précédentes, dans lequel au moins 90 % en poids du métal noble se trouvent dans les 50 % externes de volume de catalyseur.

11. Procédé selon la revendication 10, dans lequel au moins 95 % en poids du métal noble se trouvent dans les 30 % externes de volume de catalyseur.

12. Procédé selon l'une quelconque des revendications précédentes, dans lequel le support est choisi dans le groupe constitué d'alumine γ, δ ou θ, oxyde de titane, oxyde de cérium, et oxyde de zinc.

13. Procédé selon l'une quelconque des revendications précédentes, dans lequel le support comprend de l'oxyde de titane.

14. Procédé selon l'une quelconque des revendications précédentes, dans lequel le support ne comprend pas de silice non dopée ou de carbure de silicium.
